(19) **Europäisches Patentamt European Patent Office Office européen des brevets**

(11) **EP 1 997 520 B1**

(12) **EUROPEAN PATENT SPECIFICATION**

(45) Date of publication and mention
of the grant of the patent:
**24.11.2010   Bulletin 2010/47**

(51) Int Cl.:
*A61L 15/58* (2006.01)          *A61K 9/70* (2006.01)
*C09J 133/10* (2006.01)

(21) Application number: **08251906.7**

(22) Date of filing: **30.05.2008**

(54) **Patch and patch preparation**

Pflaster und Pflasterherstellung

Patch et préparation de patch

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **01.06.2007   JP 2007147112**

(43) Date of publication of application:
**03.12.2008   Bulletin 2008/49**

(73) Proprietor: **Nitto Denko Corporation
Ibaraki-shi
Osaka 567-8680 (JP)**

(72) Inventors:
• **Ameyama, Satoshi
Osaka 567-8680 (JP)**
• **Inosaka, Keigo
Osaka 567-8680 (JP)**

• **Nakamura, Kouji
Osaka 567-8680 (JP)**

(74) Representative: **Duckworth, Timothy John
J.A. Kemp & Co.
14 South Square
Gray's Inn
London WC1R 5JJ (GB)**

(56) References cited:
**EP-A- 1 591 505       EP-A- 1 716 850
EP-A- 1 925 300       WO-A-96/05813
JP-A- 2000 044 904**

Remarks:
The file contains technical information submitted after the application was filed and not included in this specification

**Description**

## TECHNICAL FIELD OF THE INVENTION

The present invention relates to a patch and a patch preparation both having an adhesive layer on at least one surface of a support.

## BACKGROUND OF THE INVENTION

In recent years, various patch preparations such as a patch applicable for the protection of the skin and the like, as well as poultice, tape preparation and the like, which are applicable for the administration of a drug into the body through skin surface and the like, have been developed, and the pertinent techniques are described, for example, in the following publications.

JP-A-10-504552 discloses a patch composed of a polyester sheet and a medical pressure sensitive adhesive containing (a) a self-adhesive polyacrylate copolymer containing copolymerized (meth)acrylic acid at a given ratio, (b) a polymer containing a basic amino group and (c) a plasticizer, which is applied on the sheet. This publication indicates that the adhesive should have a minimum level of adhesiveness, which turns into higher adhesiveness in the presence of water due to perspiration and the like.

However, this publication does not disclose vinylpyrrolidone, and does not even suggest copolymerization of vinylpyrrolidone, a monomer containing a carboxyl group and (meth)acrylic acid ester.

JP-A-2000-44904 discloses a patch preparation having an adhesive layer formed by blending a copolymer obtained by copolymerizing acid 2-ethylhexyl acrylate and acrylic acid at a given ratio (Component A), a aminoalkyl methacrylate/alkyl methacrylate copolymer (Component B), a predetermined liquid component (Component C) and a drug, and crosslinking the blend (Example 3). The publication describes that such patch preparation shows well-balanced drug solubility and adhesion to the skin.

However, this publication does not disclose or suggest containing, in an adhesive layer, a particular copolymer containing a monomer having a basic group independently of a copolymer containing vinylpyrrolidone.

In addition, both of the above-mentioned publications do not have a problem of suppressing occurrence of an adhesive residue on the skin upon peeling off of a patch in the presence of water due to perspiration and the like.

## DISCLOSURE OF THE INVENTION PROBLEMS TO BE SOLVED BY THE INVENTION

In view of the above, the present invention aims to provide a patch wherein the occurrence of an adhesive residue on the skin is suppressed upon peeling off of a patch in the presence of water due to perspiration and the like.

## MEANS OF SOLVING THE PROBLEMS

The present inventors have conducted intensive studies in an attempt to achieve the aforementioned object and found that the cohesion force of an adhesive layer can be ensured in the presence of water due to perspiration and the like by a combined use of a copolymer containing vinylpyrrolidone and a particular copolymer containing a monomer having a basic group. They have also found a new effect provided by this constitution that it surprisingly enhances even the skin adhesion force and, in a preferable embodiment, the skin adhesion force is synergistically enhanced, which resulted in the completion of the present invention.

Accordingly, the present invention provides the following:

(1) a patch comprising a support and an adhesive layer provided on at least one surface of the support, wherein the adhesive layer is formed by blending the following component (A) to component (C):

(A) a copolymer obtained by copolymerization of a monomer containing a carboxyl group, (meth)acrylic acid ester and vinylpyrrolidone as essential components;
(B) a copolymer obtained by copolymerization of a monomer having a basic group and (meth)acrylic acid ester as essential components; and
(C) a liquid component, and crosslinking the blend wherein 1.5 to 12.5 parts by weight of component (B) is blended relative to 45 parts by weight of component (A).

(2) the patch of (1), wherein the adhesive layer is crosslinked by a chemical crosslinking treatment;
(3) the patch of any of (1) to (2), wherein the basic group is at least one selected from the group consisting of an optionally substituted amino group, a pyridine group and an imidazole group; and

(4) a patch preparation comprising the patch of any of (1) to (3), wherein the adhesive layer contains a drug.

## EFFECT OF THE INVENTION

The patch of the present invention is superior in the cohesion force of an adhesive layer in the presence of water due to perspiration and the like, since it contains, in combination, a particular copolymer containing vinylpyrrolidone and a particular copolymer containing a monomer having a basic group in the adhesive layer thereof. As a result, the present invention affords a remarkable effect of suppressing cohesive failure and adhesive residue on the skin surface upon peeling off of a patch from the skin surface during sweating and the like.

In addition, since a particular copolymer containing vinylpyrrolidone and a particular copolymer containing a monomer having a basic group are used in combination in the adhesive layer, skin adhesion force is also enhanced, and an unpredictable effect of synergistic enhancement of the skin adhesion force can be achieved in a preferable embodiment.

Moreover, since the adhesive layer is efficiently crosslinked in the patch of the present invention, the adhesive layer can retain a large amount of a liquid component, thus efficiently providing a gel-like structure. Therefore, the adhesive layer affords a superior soft feeling during application to the skin and does not cause skin irritation easily during peeling.

## BRIEF DESCRIPTION OF THE DRAWINGS

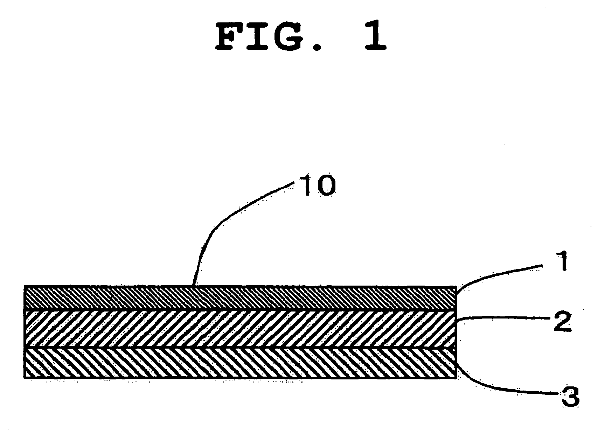

Fig. 1 is a schematic sectional view showing one embodiment of the patch or a patch preparation of the present invention.

explanation of symbols

1 support
2 adhesive layer
3 release liner
10 patch or patch preparation

## BEST MODE FOR CARRYING OUT THE INVENTION

As a support to be used in the present invention, one that does not permit a copolymer, a liquid component, a drug and the like contained in the adhesive layer to penetrate the support and be lost from the back face to decrease the content is preferable.

Specifically, a single film, a laminate film and the like, of polyester, nylon, saran, polyethylene, polypropylene, polyvinyl chloride, ethylene-ethyl acrylate copolymer, polytetrafluoroethylene, surlyn, metal foil and the like can be used.

To improve the adhesion force (anchor force) between the support and the below-mentioned adhesive layer, the support is preferably a laminate film of a non-porous plastic film and a porous film, both composed of the above-mentioned materials. In this case, the adhesive layer is preferably formed on the porous film side.

As the porous film, a film capable of improving the anchor force to the adhesive layer is employed. Specifically, paper, woven fabric, non-woven fabric, mechanically perforated sheet and the like are used. Among these, paper, woven fabric and non-woven fabric are particularly preferable from the aspects of handleability and the like.

In the patch of the present invention, an adhesive layer formed on at least one surface of the above-mentioned support contains particular copolymer component (A), component (B) and liquid component (C) as essential components, and has a crosslinked structure with suitable elasticity achieved by crosslinking. In other words, it has what is called a gel-like structure.

First, the copolymer for the above-mentioned component (A) can be obtained by copolymerizing a monomer containing a carboxyl group, vinylpyrrolidone and (meth)acrylic acid ester as essential components. The copolymer for component (A) is mainly used as a component for an adhesive layer to improve adhesiveness and compatibility with the liquid component to be added.

Examples of the monomer containing a carboxyl group for the copolymer of the above-mentioned component (A) include (meth)acrylic acid, itaconic acid, maleic acid, maleic anhydride and the like. These monomers can be used alone or as a blend of two or more kinds thereof. From the aspects of reactivity and the adhesiveness of the finished tape, acrylic acid is preferable. The proportion of these monomers is preferably 1 - 20 wt%, more preferably 1 - 10 wt%, of the copolymer of component (A), from the aspects of adhesiveness and cohesion as adhesive properties, drug releaseability when a drug is contained in an adhesive layer, reactivity during crosslinking treatment of an adhesive layer and the like.

As vinylpyrrolidone in the copolymer of the above-mentioned component (A), N-vinyl-2-pyrrolidone, methylvinylpyrrolidone and the like can be used, which may be used alone or as a blend of two or more kinds thereof. From the aspect of reactivity, N-vinyl-2-pyrrolidone is preferable. From the aspect of cohesion force, the proportion thereof is preferably 10 - 30 wt%, more preferably 19 - 30 wt%, of the copolymer of component (A).

While (meth)acrylic acid ester in the copolymer of the above-mentioned component (A) is not particularly limited, from the aspects of adhesiveness and the like, (meth)acrylic acid alkyl ester having an alkyl group having a carbon number of not less than 4 is preferably used. Specifically, (meth)acrylic acid alkyl ester having a straight or branched chain alkyl group having a carbon number of 4 to 13, such as butyl, pentyl, hexyl, heptyl, octyl, nonyl, decyl, undecyl, dodecyl, tridecyl and the like, and the like, can be used, which may be used alone or as a blend of two or more kinds thereof.

From the aspect of adhesiveness at ambient temperature, (meth)acrylic acid alkyl ester wherein the alkyl group is butyl, 2-ethylhexyl or cyclohexyl is more preferable, and (meth)acrylic acid alkyl ester wherein the alkyl group is 2-ethylhexyl is most preferable. From the aspect of adhesiveness, the proportion of these monomers can be set to any level as long as it falls within the range of preferably 30 - 89 wt%, more preferably 60 - 80 wt%, of the copolymer of component (A).

The above-mentioned (meth)acrylic acid alkyl ester is not limited to those recited above as examples, and it is obvious that an ester compound of a group other than alkyl group, (meth)acrylic acid alkyl ester having an alkyl group with a carbon number of 1 to 3 and (meth)acrylic acid alkyl ester having an alkyl group with a carbon number of not less than 14 may be used in combination, as long as the effect of the present invention can be exhibited.

In the present invention, the copolymer of component (B) is used as a component for improving the adhesiveness of an adhesive layer, and further, improving drug solubility when an adhesive layer contains a drug.

Examples of the monomer having a basic group as the copolymer of the above-mentioned component (B) include acrylic monomers, vinyl monomers and the like, which have an optionally substituted amino group (particularly alkylamino group), pyridine group, imidazole group and the like as a side chain. More specifically, mono or dialkylamino (meth)acrylate having an alkyl group with a carbon number of 1 to 4, such as aminoethyl(meth)acrylate, dimethylaminoethyl(meth)acrylate, dimethylaminopropyl(meth)acrylate, dimethylaminobutyl(meth)acrylate and the like, vinylpyridine, vinylimidazole and the like. From the aspect of handleability such as reactive and the like, the monomer having a basic group basic group is preferably dimethylaminoethyl(meth)acrylate. These monomers having a basic group can be used alone or as a blend of two or more kinds thereof. The proportion of these monomers is preferably 20 - 70 wt%, more preferably 30 - 60 wt%, of the copolymer of component (B), from the aspects of compatibility with the copolymer of component (A), adhesive property to be maintained and the like.

The kind of the (meth)acrylic acid ester as the above-mentioned copolymer of component (B) is the same as that recited for the aforementioned component (A), which may be used alone or as a blend of two or more kinds thereof. The proportion of these monomers is preferably 30 - 80 wt%, more preferably 40 - 70 wt%, of the copolymer of component (B), from the aspects of compatibility with the copolymer of component (A), adhesive property to be maintained and the like.

The copolymers of the above-mentioned component (A) and component (B) may be each copolymerized with any amount of other copolymerizable monomers as necessary, as long as the property of each component in the present invention can be maintained.

Among the copolymers of the above-mentioned component (A) and component (B), most preferred as the copolymer of component (A) is a copolymer obtained by copolymerizing 60 - 80 wt% of 2-ethylhexyl acrylate, 19 - 30 wt% of N-vinyl-2-pyrrolidone, and 1 - 10 wt% of acrylic acid as essential components, most preferred as the copolymer of component (B) is a copolymer obtained by copolymerizing 30 - 60 wt% of dimethylaminoethyl methacrylate with 15 - 40 wt%, preferably 20 - 40 wt%, of methyl methacrylate and 15 - 40 wt%, preferably 20 - 40 wt%, of butyl methacrylate as essential components, and the like, in view of comfortableness during adhesion and the balance of drug solubility when an adhesive layer contains a drug.

While the proportion of the total weight of component (A) and component (B) relative to the total weight of the adhesive layer is not particularly limited, it is preferably 30 - 65 wt%, more preferably 35 - 60 wt%, of the total weight of the adhesive layer to impart sufficient adhesiveness to an adhesive layer. As regards the proportion of component (A) and component (B), 1.5 - 12.5 parts by weight, more preferably 1.5 - 7.5 parts by weight, of component (B) relative to 45 parts by weight of component (A) is blended. When component (B) is less than 1 part by weight, adhesion force becomes insufficient. When a drug is contained, moreover, the solubility of the drug may become insufficient. When the proportion exceeds 20 parts by weight, adhesion force becomes too strong, possibly causing skin irritation upon peeling off. Furthermore, component (B) is blended in a proportion of 1.5 - 12.5 parts by weight, more preferably 1.5 - 7.5 parts by weight, relative to 45 parts by weight of component (A), for expression of a synergistic effect on the adhesion force.

The liquid component as component (C) to be contained in an adhesive layer together with the above-mentioned component (A) and component (B) in the present invention is uniformly dissolved and dispersed in the adhesive layer, plasticizes the crosslinked adhesive layer into a gel-like state, and achieves a soft feeling thereof. In other words, pain and skin irritation due to an adhesive force (skin adhesion force), which is produced when an acrylic adhesive tape and a transdermal absorption preparation of the present invention are peeled off from the skin surface, can be reduced in the present invention by adding component (C) to cause crosslinking gellation. Moreover, since an adhesive layer is plasticized as mentioned above, when a drug is contained as a patch preparation, the drug acquires good free diffusability, which in turn improves releaseability onto the skin surface (transfer to the skin).

Component (C) is not particularly limited as long as it has compatibility with the above-mentioned component (A) and component (B), and has a plasticizing action on an adhesive layer. From the aspect of the compatibility, an organic liquid component is preferable. Examples thereof include glycols such as ethylene glycol, diethylene glycol, triethylene glycol, propylene glycol, polyethylene glycol, polypropylene glycol and the like, fats and oils such as olive oil, castor oil, squalene, lanolin and the like, organic solvents such as ethyl acetate, ethyl alcohol, dimethyldecyl sulfoxide, methyloctyl sulfoxide, dimethyl sulfoxide, dimethylformamide, dimethylacetamide, dodecylpyrrolidone, isosorbitol and the like, liquid surfactant, plasticizers such as tributyl acetylcitrate, diisopropyladipate, phthalic acid ester, diethyl sebacate, triethyl citrate, tributyl acetylcitrate and the like, hydrocarbons such as liquid paraffin and the like, ethoxylated stearyl alcohol, glycerol fatty acid ester, isopropyl myristate, isotridecyl myristate, ethyl laurate, N-methylpyrrolidone, ethyl oleate, oleic acid, diisopropyl adipate, isopropyl palmitate, 1,3-butanediol and the like. These may be used alone or as a blend of two or more kinds thereof.

Of the liquid components of the above-mentioned component (C), preferable organic liquid components include fatty acid ester, glycerol fatty acid ester (particularly monoglyceride), tributyl acetylate can be mentioned.

These fatty acid esters and glycerol fatty acid esters may by any as long as they exhibit an adhesive layer plasticizing action. When they comprise a fatty acid having an unnecessarily large or small number of carbons, the aforementioned compatibility with acrylic copolymers may be degraded or the acid may be volatilized during a heating step for preparation of a patch.

In addition, when they comprise a fatty acid having a double bond in a molecule, oxidative decomposition and the like may be developed to cause problems in the preservation stability. In the case of the below-mentioned patch preparation, moreover, high drug contents per unit area may result in crystallization of drugs in the preparation as they exceed the saturation solubility. Certain kind of fatty acid esters and glycerol fatty acid esters to be added may inhibit crystallization of the drug or delay the precipitation speed, thus sometimes degrading the appearance of the obtained preparation or adversely affecting the preservation stability.

As the fatty acid ester to be used, therefore, a fatty acid ester composed of higher fatty acid preferably having a carbon number of 12 - 16, more preferably 12 - 14, and lower monadic alcohol preferably having a carbon number of 1 - 4 is preferably employed.

Examples of the higher fatty acid preferably include lauric acid (C12), myristic acid (C14) and palmitic acid (C16), particularly myristic acid. Examples of the lower monadic alcohol include methyl alcohol, ethyl alcohol, propyl alcohol and butyl alcohol. They are not limited to straight chain alcohols and may be branched alcohols. Preferably, isopropyl alcohol is used. Accordingly, the most preferable fatty acid ester is isopropyl myristate.

On the other hand, as the glycerol fatty acid ester, glycerides composed of higher fatty acid having a carbon number of 8 - 10 and glycerol is preferable. Examples of the higher fatty acid preferably include caprylic acid (octanoic acid, C8), pelargonic acid (nonanoic acid, C9) and capric acid (decanoic acid, C10), particularly caprylic acid monoglycerides, diglycerides and triglyceride using caprylic acid.

The proportion of component (C) to be blended is preferably 0.5 - 1.5 parts by weight, more preferably 0.7 - 1.5 parts by weight, per 1 part by weight of the total amount of the above-mentioned component (A) and component (B). When the content of component (C) is outside these ranges, practical skin adhesiveness and low skin irritation may not be achieved and, in the below-mentioned patch preparation, drug release performance (skin transfer performance) may be insufficient.

The adhesive layer in the patch of the present invention contains the above-mentioned component (A), component (B) and component (C) as essential components. When these components are to be solved or mixed, they need to be compatible with each other. Thus, a mixed solvent of an amphiphilic solvent compatible with both water and oil, such as isopropyl alcohol, tetrahydrofuran, acetone and the like, and a hydrophobic solvent, such as ethyl acetate and the like, that dissolves a copolymer, is preferably used for mixing.

In the present invention, blending is performed as mentioned above, and an adhesive layer is crosslinked to form what is called a gel-like state, thereby suppressing diffluence of the contained liquid component, and further conferring a cohesion force to the adhesive layer.

Crosslinking is performed by physical crosslinking by exposure to radiation such as UV irradiation, electron beam irradiation and the like, chemical crosslinking and the like. To reduce an influence on adhesive layer components, chemical crosslinking is preferable. Specifically, chemical crosslinking using crosslinking agents such as polyisocyanate compounds, organic peroxides, organic metal salts, metal alcoholates, metal chelate compounds, multifunctional compounds and the like, and the like are employed.

Of these crosslinking agents, to effectively ensure the cohesion force of an adhesive layer in the presence of water, a chelate compound, particularly, an aluminum chelate compound, is preferable. Such crosslinking agents are free of a thickening phenomenon of the solution up to coating and drying, and is extremely superior in workability. The amount of the crosslinking agent to be blended in this case is preferably about 0.01 - 2 parts by weight relative to 100 parts by weight of the total weight of the polymer of component (A).

Since the patch preparation of the present invention protects the adhesive face of an adhesive layer before use, a release liner is preferably laminated on the adhesive face. The release liner is not particularly limited as long as it ensures sufficient easy-to-release property. Examples thereof include films of polyester, polyvinyl chloride, polyvinylidene chloride, poly(ethylene terephthalate) and the like, laminate films of polyolefin and paper such as high quality paper, glassine paper and the like, high quality paper, glassine paper and the like, and the like, whose face to be in contact with an adhesive layer underwent a peeling treatment by applying a silicone resin, a fluororesin and the like. The thickness of the release liner is generally 10 - 200 $\mu$m, preferably 25 - 100 $\mu$m.

As the release liner in the present invention, one made of a polyester (particularly, poly(ethylene terephthalate)) resin is preferable from the aspects of barrier property and cost. In this case, one having a thickness of about 25 - 100 $\mu$m is more preferable in view of handleability.

A patch preparation can be obtained by impregnating an adhesive layer of a patch as mentioned above with a drug. The drug here is not particularly limited, but a drug that can be administered to a mammal such as human and the like through the skin, i.e., transdermally absorbable drug, is preferable. Where necessary, two or more kinds of drugs may be contained.

While the content of the drugs can be appropriately determined based on the kind of the drug and object of administration, it is preferably within the range of about 1 - 40 wt%, more preferably about 3 - 30 wt%, relative to the total weight of an adhesive layer. When the content is less than 1 wt%, release of an amount effective for the treatment or prophylaxis may not be expected, and when it exceeds 40 wt%, enhancement of the effect cannot be expected due to the increased weight, which is economically disadvantageous as well as inferior in the adhesiveness to the skin.

The patch of the present invention and patch preparation is produced, for example, in the following manner: starting materials such as copolymers, a liquid component and, where necessary, a drug, a crosslinking agent and the like are dissolved or dispersed in a solvent, the obtained solution or dispersion is applied on at least one surface of a support, which is dried to form an adhesive layer on the surface of the support, and a release liner is formed. Alternatively, the above-mentioned solution or dispersion is applied on at least one surface of a release liner for protection, which is dried to form an adhesive layer on the surface of the release liner, and a support is adhered to the adhesive layer. It is preferable to further include a curing step to promote crosslinking of the adhesive layer.

**Examples**

The present invention is explained in more detail in the following by referring to Examples, which are not to be construed as limitative. In the following description, parts and % mean parts by weight and wt%, respectively.

(Preparation of copolymer solution)

As component (A), 2-ethylhexyl acrylate (72 wt%), N-vinyl-2-pyrrolidone (25 wt%), and acrylic acid (3 wt%) were copolymerized to give acrylic copolymer (a). As Comparative Example, as an acrylic copolymer without vinylpyrrolidone, 2-ethylhexyl acrylate (95 wt%) and acrylic acid (5 wt%) were copolymerized to give acrylic copolymer (b). As component (B), 50 wt% of dimethylaminoethyl methacrylate, 25 wt% of methyl methacrylate and 25 wt% of butyl methacrylate were copolymerized to give a copolymer.

1. Patch

(Examples 1 - 4)

To a solution of acrylic copolymer (a) (solid content 45 parts) as component (A) were added a solution of the above-mentioned component (B) in an amount shown in Table 1, isopropyl myristate (hereinafter "IPM") as component (C) in an amount shown in Table 1, ethylacetoacetatealuminum diisopropylate as a crosslinking agent, and isopropanol and ethyl acetate in an adequate amount for adjusting the concentration, and the mixture was stirred in a high-speed mixer to give a uniform adhesive solution. The crosslinking agent was added in the proportion of 0.3 part per 100 parts of the solid content of acrylic copolymer (a). The obtained adhesive solution was applied to a 75 $\mu$m-thick polyester release sheet such that the thickness after drying would be 80 $\mu$m, and dried to give a crosslinked gel-like adhesive layer. The adhesive layer prepared above was laminated on the surface of a non-woven fabric of a support prepared by adhesion laminating a polyester non-woven fabric (8 g/m$^2$ fabric weight) on one surface of a 2 $\mu$m-thick polyester film, and the laminate was aged with heating at 60°C for 48 hr to give a patch of the present invention.

(Comparative Example 1)

In the same manner as in Examples 1 to 4 except that component (B) was not blended and IPM as component (C) was blended in the amount shown in Table 1, a patch of Comparative Example 1 was prepared.

(Comparative Examples 2 - 4)

In the same manner as in Examples except that a solution of acrylic copolymer (b) (solid content 55 parts) was used instead of a solution of acrylic copolymer (a) (solid content 45 parts) as component (A), patches of Comparative Examples 2 to 4 were prepared. Here, the amount of acrylic copolymer (b) to be blended was determined in such a manner that Comparative Example 1 using acrylic copolymer (a) and not using component (B) and Comparative Example 4 using acrylic copolymer (b) and not using component (B) would have almost the same adhesive force. The crosslinking agent was added in a proportion of 0.3 part relative to 100 parts of the solid content of acrylic copolymer (b).

(Measurement method of evaluation values)

<Cohesion force of adhesive layer in the presence of water>

A sample was adhered onto the inner upper arm of 5 volunteers, and peeled off 1 to 2 hours after perspiration or bathing. Adhesive residue at that time was evaluated based on the following criteria and averaged.

Criteria:

1 No adhesive residue
2 Slight adhesive residue
3 Clear adhesive residue

The adhesive layer of a sample with a large amount of adhesive residue was evaluated to have low cohesion force in the presence of water.

<Adhesive force>

A band-like strip (width 24 mm) of each preparation sample was adhered to a bakelite board, pressed by one reciprocation of a roller with a load of 300 g and peeled off in a 180 degree direction at a rate of 300 mm/min, and the adhesive force (release force) was measured.

<Gel fraction>

Each sample was cut into 10 cm$^2$, and the weight ($W_1$) of the adhesive layer was measured. The sample was immersed in 100 ml of ethyl acetate for 24 hr, and ethyl acetate was exchanged. This operation was repeated three times and the solvent-soluble part was extracted. The sample was taken out and dried, and the weight ($W_2$) of the adhesive layer was measured, based on which the gel fraction was calculated from the following formula.

$$\text{Gel fraction (\%)} = (W_2 \times 100)/(W_1 \times A/B)$$

A=weight of (component (A)+crosslinking agent),
B=weight of (component (A)+component (B)+component
(C)+(drug)(if contained)+crosslinking agent)
High gel fraction was evaluated to show sufficient crosslinking of the adhesive layer.

<Skin irritation >

An estimator adhered a preparation for evaluation (about 10 cm$^2$), and evaluated the presence or absence of irritation upon peeling off two minutes later based on the following criteria.

Criteria:

◎ No skin irritation
○ Almost no skin irritation
△ Slight skin irritation
x Strong skin irritation

The results are shown in Table 1.

Table 1

| | | component (A) (solid content, parts) | component (B) (solid content, parts) | component (C) IPM (parts) | cohesion force of adhesive layer in the presence of water | adhesive force (N/24 mm) | gel fraction (%) | skin irritation |
|---|---|---|---|---|---|---|---|---|
| Example 1 | acrylic copolymer (a) 45 | 15 | 40 | 1 | 3.1 | 102 | △ |
| Example 2 | | 10 | 45 | 1 | 2.6 | 99 | ○ |
| Example 3 | | 5 | 50 | 1 | 2.0 | 89 | ◎ |
| Example 4 | | 2 | 53 | 1 | 1.8 | 84 | ◎ |
| Comparative Example 1 | | | 55 | 1 | 1.1 | 78 | ◎ |
| Comparative Example 2 | acrylic copolymer (b) 55 | 5 | 40 | 3 | 1.6 | 88 | ◎ |
| Comparative Example 3 | | 2 | 43 | 2 | 1.4 | 97 | ◎ |
| Comparative Example 4 | | 0 | 45 | 1 | 1.0 | 79 | ◎ |

As is clear from Table 1, the patches of the Examples showed high cohesion force, high adhesive force and high gel fraction of the adhesive layer in the presence of water. In contrast, Comparative Example 1 and Comparative Example 4 showed high cohesion force of an adhesive layer in the presence of water but low adhesive force and low gel fraction. In addition, Comparative Example 2 and Comparative Example 3 showed markedly degraded cohesion force of the adhesive layer in the presence of water, and adhesive residue was produced upon peeling off.

Example 3 showed an adhesive force exceeding the arithmetic average of Comparative Example 1 and Comparative Example 2, and Example 4 showed an adhesive force exceeding the arithmetic average of Comparative Example 1 and Comparative Example 3. This suggests a synergistically enhanced adhesive force of the patch of the present invention. Moreover, Examples 3 and 4 showed ideal results with respect to skin irritation.

In Example 1 where 15 parts of component (B) was blended relative to 45 parts of component (A), skin irritation was observed somewhat. Since Example 2 using 10 parts of component (B) hardly showed skin irritation, and Examples 3 and 4 clearly showed synergistic enhancement of the adhesive force, it has been shown that the proportion of 1.5 - 12.5 parts by weight of component (B) relative to 45 parts of component (A) is preferable and 1.5 - 7.5 parts by weight is more preferable. 2. Patch preparation

In the same manner as in Examples 1 - 4 except that 1 part of 40 - 53 parts of IPM as component (C) is replaced by a drug (indomethacin), a patch preparation of the present invention is prepared. The patch of the present invention preparation shows less adhesive residue upon peeling, like the patch of the present invention, and has a sufficient skin adhesive force.

**Claims**

1. A patch comprising a support and an adhesive layer provided on at least one surface of the support, wherein the adhesive layer is formed by blending the following component (A) to component (C):

   (A) a copolymer obtainable by copolymerization of a monomer containing a carboxyl group, (meth)acrylic acid ester and vinylpyrrolidone as essential components;
   (B) a copolymer obtainable by copolymerization of a monomer having a basic group and (meth)acrylic acid ester as essential components; and
   (C) a liquid component, and

   crosslinking the blend, wherein 1.5-12.5 parts by weight of component (B) is blended relative to 45 parts by weight of component (A).

2. A patch according to claim 1, wherein the adhesive layer is crosslinked by a chemical crosslinking treatment.

3. A patch according to claim 1 or claim 2, wherein the basic group is at least one selected from the group consisting of an optionally substituted amino group, a pyridine group and an imidazole group.

4. A patch preparation comprising a patch according to any one of claims 1 to 3, wherein the adhesive layer comprises a drug.


**Patentansprüche**

1. Pflaster, das einen Träger und eine auf wenigstens einer Fläche des Trägers angeordnete Klebeschicht umfasst, wobei die Klebeschicht gebildet wird durch Mischen der folgenden Komponente (A) mit Komponente (C):

   (A) ein Copolymer, das durch Copolymerisation eines Monomers, das eine Carboxygruppe enthält, von (Meth) acrylsäureester und Vinylpyrrolidon als wesentliche Komponenten erhältlich ist;
   (B) ein Copolymer, das durch Copolymerisation eines Monomers, das eine basische Gruppe aufweist, und von (Meth)acrylsäureester als wesentliche Komponenten erhältlich ist; und
   (C) eine flüssige Komponente; und

   Vernetzen des Gemischs, wobei 1,5 bis 12,5 Gewichtsteile Komponente (B) auf 45 Gewichtsteile Komponente (A) gemischt werden.

2. Pflaster gemäß Anspruch 1, wobei die Klebeschicht durch eine chemische Vernetzungsbehandlung vernetzt wird.

3. Pflaster gemäß Anspruch 1 oder 2, wobei die basische Gruppe wenigstens eine ist, die aus der Gruppe ausgewählt ist, die aus einer gegebenenfalls substituierten Aminogruppe, einer Pyridingruppe und einer Imidazolgruppe besteht.

4. Pflasterpräparat, das ein Pflaster gemäß einem der Ansprüche 1 bis 3 umfasst, wobei die Klebeschicht einen Wirkstoff umfasst.


**Revendications**

1. Timbre comprenant un support et une couche adhésive disposée sur au moins une surface du support, dans lequel la couche adhésive est formée en mélangeant le composant (A) au composant (C) suivantes :

   (A) un copolymère pouvant être obtenu par copolymérisation d'un monomère contenant un groupe carboxyle, un ester d'acide (méth)acrylique et de la vinylpyrrolidone comme composants essentiels ;
   (B) un copolymère pouvant être obtenu par copolymérisation d'un monomère ayant un groupe basique et un ester d'acide (méth)acrylique comme composants essentiels ; et
   (C) un composant liquide, et

   en réticulant le mélange, où 1,5 à 12,5 parties en poids de composant (B) sont mélangés à 45 parties en poids de composant (A).

2. Timbre selon la revendication 1, dans lequel la couche adhésive est réticulée par un traitement de réticulation chimique.

3. Timbre selon la revendication 1 ou la revendication 2, dans lequel le groupe basique est au moins un élément choisi dans le groupe consistant en un groupe amino, un groupe pyridine et un groupe imidazole facultativement substitués.

4. Préparation de timbre comprenant un timbre selon l'une quelconque des revendications 1 à 3, dans laquelle la couche adhésive comprend un médicament.

# FIG. 1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10504552 A

- JP 2000044904 A